# EUROPÄISCHE PATENTSCHRIFT

(11) **EP 1 242 445 B1**
(45) Veröffentlichungstag und Bekanntmachung des Hinweises auf die Patenterteilung: **23.04.2003**
(21) Anmeldenummer: 00988735.7
(22) Anmeldetag: 21.11.2000
(51) Int. Cl.: C07J 53/00, A61K 31/567, A61P 35/00

(54) **14,15-CYCLOPROPANOSTEROIDE DER 19-NORANDROSTAN-REIHE, VERFAHREN ZU IHRER HERSTELLUNG UND DIESE VERBINDUNGEN ENTHALTENDE PHARMAZEUTISCHE ZUSAMMENSETZUNGEN**
14,15-CYCLOPROPANOSTEROIDS OF THE 19-NORANDROSTANE SERIES, METHOD FOR PRODUCING SAID COMPOUNDS AND PHARMACEUTICAL PREPARATION CONTAINING SAID COMPOUNDS
14,15-CYCLOPROPANOSTEROIDES DE LA SERIE DES 19-NORANDROSTANES, PROCEDE POUR LEUR PRODUCTION ET PREPARATIONS PHARMACEUTIQUES CONTENANT CES COMPOSES

(30) Priorität: 08.12.1999 DE 19959697
(43) Veröffentlichungstag der Anmeldung: 25.09.2002
(73) Patentinhaber: Schering Aktiengesellschaft, 13353 Berlin (DE)
(72) Erfinder: RING, Sven, 07749 Jena (DE); ELGER, Walter, 14195 Berlin (DE); KAUFMANN, Günter, 07743 Jena (DE)
(74) Vertreter: Ziebig, Marlene, Dr. Dipl.-Chem.
(86) Internationale Anmeldenummer: EP0011553
(87) Internationale Veröffentlichungsnummer: WO01042274

(56) Entgegenhaltungen:
- EP-A- 0 768 316
- DE-A- 19 827 522

## Beschreibung

Die Erfindung betrifft neue 14,15-Cyclopropanosteroide der 19-Norandrostan-Reihe, deren Herstellung und diese Verbindungen enthaltende pharmazeutische Zusammensetzungen.
14,15-Cyclopropanosteroide der 19-Norandrostan-Reihe der folgenden Formel 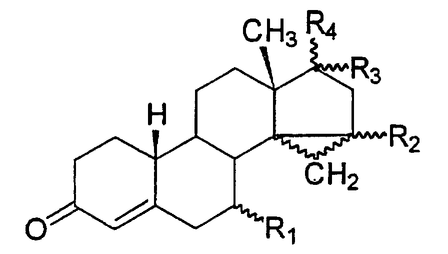 sind in der deutschen Anmeldung Nr. 198 27 522.6 (PCT/DE99/01795) beschrieben, die einen früheren Zeitrang als die vorliegende Anmeldung beansprucht, aber nach deren Einreichung veröffentlicht wird.

In der Formel ist R₁ ein Wasserstoffatom oder ein Alkylrest mit 1 bis 9 Kohlenstoffatomen,
steht R₂ für ein Wasserstoffatom oder eine Methylgruppe,
stehen R₃ und R₄ unabhängig voneinander für ein Wasserstoffatom,
für eine Hydroxygruppe, für eine Acyloxygruppe -O-CO-R₅ mit R₅ für 1 bis 10 Kohlenstoffatome,
für eine Carbamoyloxygruppe O-CO-NHR₆ mit R₆ für ein Wasserstoffatom, einen Alkyl-, Arylrest mit jeweils 1 bis 5 Kohlenstoffatomen,
für eine Sulfamoyloxygruppe -O-SO₂-NR₇R₈ mit R₇ und R₈ unabhängig voneinander jeweils für ein Wasserstoffatom, eine Alkyfgruppe mit 1 bis 5 Kohlenstoffatomen oder zusammen mit dem Stickstoffatom für eine Pyrrolidino-, Piperidino- oder Morpholinogruppe,
für eine Gruppierung -CH₂R₉ mit R₉ für eine Hydroxygruppe, eine Alkyloxygruppe mit 1 bis 5 Kohlenstoffatomen, ein Chlor- oder Bromatom, eine Azid-, Nitrilo- oder Thiocyangruppe oder für eine Gruppierung -SR₁₀ mit R₁₀ für eine Alkygruppe mit 1 bis 5 Kohlenstoffatomen,
oder stehen R₃ und R₄ zusammen für eine Oxogruppe,
oder bilden R₃ und R₄ unter Einschluß des C-17 ein Spirooxiran oder ein 2,2-Dimethyl-1,3-dioxolan und ist der 14,15-Cyclopropanring entweder α- oder β-ständig angeordnet.

Aus EP 0 768 316 A1 sind Steroide mit einer 14,15-Methylengruppe bekannt, die progesterone Wirkung aufweisen und damit in Kombination mit mindestens einem geeigneten Estrogen zur hormonalen Kontrazeption und in der klimakterischen Hormon-Replacement-Therapie (HRT) sowie zur Behandlung der Endometriose oder gestagenabhängiger Tumore geeignet sind.

Gegenüber diesem Stand der Technik liegt der vorliegenden Erfindung die Aufgabe zugrunde, neue ungesättigte 14,15-Cyclopropano-Androstane, bereitzustellen.

Diese Aufgabe wird durch 14,15-Cyclopropanosteroide der 19-Norandrostan-Reihe der allgemeinen Formel (I) 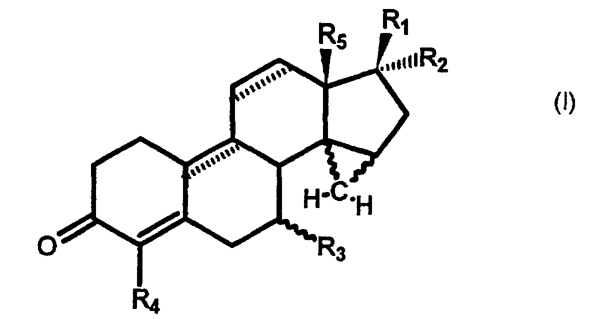
worin R₁ ein Wasserstoffatom, eine Hydroxygruppe, eine C₁₋₁₀-Alkyl-, C₁₋₁₀-Alkyloxy-, C₁-₁₅-Acyloxy-, C₄₋₁₅-Aryloxy-, C₇₋₁₅-Aralkyloxy- oder eine C₇₋₁₅-Alkylaryloxygruppe ist,
R₂ für ein Wasserstoffatom, eine Hydroxygruppe, eine C₁₋₁₀-Alkyl-, C₁₋₁₀-Acyl-, C₁₋₁₀-Acyloxy-, C₆₋₁₅-Aryl-, C₇₋₁₅-Aralkyl- oder C₇₋₁₅-Alkylarylgruppe,
für einen Rest -(CH₂)ₙCH₂Y
mit n = 0, 1 oder 2 und Y für ein Halogenatom, insbesondere ein Fluor-, Chlor-, Bromoder Jodatom, für ein Pseudohalogen, insbesondere eine Cyan-, Azid- oder Rhodanidgruppe,
für einen Rest -(CH₂)m-CH=CH(CH₂)p-R₆
mit m = 0, 1, 2 oder 3 und p = 0, 1 oder 2 und R₆ für ein Wasserstoffatom, einen C₁₋₁₀-Alkyl-, C₆₋₁₅-Aryl-, C₇₋₁₅-Aralkyl- oder C₇₋₁₅-Alkylarylrest oder eine Hydroxylgruppe, eine C₁₋₁₀-Alkyloxygruppe oder eine C₁₋₁₀-Acyloxygruppe,
für einen Rest -(CH₂)oC≡CR₇
mit o = 0, 1 oder 2 und R₇ für ein Wasserstoffatom, ein Halogenatom, insbesondere ein Fluor-, Chlor-, Brom- oder lodatom, einen C₁₋₁₀-Alkyl-, C₆₋₁₅-Aryl-, C₇₋₁₅-Aralkyl-, C₇₋₁₅-Alkylaryl- oder C₁₋₁₀-Acylrest steht,
R₁ und R₂ zusammen für eine Keto-, Methylen-, Difluormethylengruppe stehen oder unter Einschluß des C-17 ein Spirooxiran oder ein 2,2-Dimethyl-1,3-dioxolan bilden,
R₃ für ein Wasserstoffatom oder eine α- oder β-ständige C₁₋₁₀-Alkylgruppe steht,
R₄ für ein Halogenatom, insbesondere ein Fluor-, Chlor- oder Bromatom, oder ein Pseudohalogen, insbesondere eine Rhodanid- oder Azidgruppe, oder eine Hydroxyloder Perfluoralkylgruppe steht und
R₅ für eine C₁₋₄-Alkylgruppe steht, sich zwischen C-14 und C-15 eine α- oder β-Cyclopropangruppe befindet,
sich in 9,10- oder 11,12-Stellung Doppelbindungen befinden können,
mit der Maßgabe, daß, wenn sich in 9,10- und 11,12-Stellung zwei weitere Doppelbindungen befinden oder sich in 11,12-Stellung eine Doppelbindung befindet, R₄ zusätzlich zu den voranstehend genannten Bedeutungen ein Wasserstoffatom sein kann, und mit der weiteren Maßgabe, daß, wenn R₅ keine Methylgruppe ist, R₄ zusätzlich zu den voranstehend genannten Bedeutungen ein Wasserstoffatom sein kann, sowie deren pharmazeutisch verträgliche Salze gelöst.

Die Verbindungen der Formel (I) zeigen überraschenderweise ein interessantes Androgen/Gestagen-Mischprofil, wobei je nach Substitution die androgene oder auch die gestagene Aktivität überwiegen kann.

Im Sinne der Erfindung sind pharmazeutisch verträgliche Salze Alkali- oder Erdalkalisalze, insbesondere Natrium-, Kalium- oder Ammoniumsalze. Diese Salze können nach im Stand der Technik wohlbekannten Standardtechniken und -verfahren hergestellt werden.

Unter "C₁₋₄- bzw. C₁₋₁₀-Alkylgruppe" wird im Sinne der vorliegenden Erfindung ein verzweigter oder geradkettiger Alkylrest mit 1 bis 4 bzw. 1 bis 10 Kohlenstoffatomen verstanden. Als Beispiele seien eine Methyl-, Ethyl-, n-Propyl-, i-Propyl-, n-Butyl-, i-Butyloder tert.-Butyl-, n-Pentyl-, i-Pentyl-, n-Hexyl-, 2-Methylpentyl-, 3-Methylpentyl-, 2,2-Dimethylbutyl- oder 2,3-Dimethylbutylgruppe genannt.

Unter dem Begriff "C₁₋₁₀-Alkyloxygruppe" wird im Sinne der vorliegenden Anmeldung eine cyclische oder acyclische Gruppe, deren Alkylrest 1 bis 10 Kohlenstoffatome aufweist, verstanden, wobei unter einer "cyclischen Gruppe" auch eine heterocyclische Gruppe verstanden wird, die im Ring ein oder zwei Heteroatome aufweisen kann, die aus einem Stickstoffatom, einem Sauerstoffatom und einem Schwefelatom ausgewählt werden können. Beispiele sind eine Methoxygruppe, Ethoxygruppe oder n- oder iso-Propoxygruppe, iso- oder tert.-Butoxygruppe, eine (1'-Methoxy)cyclopentoxy- bzw. Tetrahydropyranyloxygruppe.

Unter dem Begriff "C₁₋₁₀- bzw. C₁₋₁₅-Acyl- bzw. Acyloxygruppe" wird im Sinne der vorliegenden Anmeldung ein Rest mit 1 bis 10 bzw. 1 bis 15 Kohlenstoffatomen der geradkettigen oder verzweigten Alkancarbonsäuren, wie beispielsweise der Ameisensäure, Essigsäure, Propionsäure, Butansäure, iso-Butansäure, Heptansäure oder Undecansäure, verstanden.

Unter dem Begriff "C₆₋₁₅-Arylgruppe" wird im Sinne der vorliegenden Anmeldung ein substituierter oder unsubstituierter Arylrest mit 6 bis 15 Kohlenstoffatomen, beispielsweise eine Phenylgruppe, eine substituierte Phenylgruppe, wie eine Halogenphenylgruppe oder eine Nitrophenylgruppe, oder eine Naphtylgruppe verstanden.

Unter dem Begriff "C₄₋₁₅-Aryloxygruppe" wird im Sinne der vorliegenden Anmeldung ein carbocyclischer oder heterocyclischer Rest mit 4 bis 15 Kohlenstoffatomen verstanden. Beispiele sind eine Benzoyloxygruppe, 1- oder 2-Naphtinyloxygruppe, 2- oder 3-Furanyloxygruppe, 2- oder 3-Thienyloxygruppe, 2-, 3- oder 4-Pyridinyloxygruppe.

Unter dem Begriff "C₇₋₁₅-Alkylarylgruppe" wird im Sinne der vorliegenden Anmeldung ein mit einem Alkylrest substituierter Arylrest, die zusammen 7 bis 15 Kohlenstoffatomen aufweisen, verstanden, wobei der Arylrest weitere Substituenten, wie beispielsweise ein Halogenatom tragen kann. Beispiele sind eine Toluenylgruppe (Methylphenylgruppe),

Halogentoluenylgruppe, Ethylphenylgruppe, Dimethylphenylgruppe oder Trimethylphenylgruppe.

Unter dem Begriff "C₇₋₁₅-Alkylaryloxygruppe" wird im Sinne der vorliegenden Anmeldung eine um ein Sauerstoffatom verlängerte "C₇₋₁₅-Aralkylgruppe", wie beispielsweise eine 3-bzw. 4-Methylphenyloxygruppe, verstanden.

Unter dem Begriff "C₇₋₁₅-Aralkylgruppe" wird im Sinne der vorliegenden Anmeldung ein mit einem Arylrest substituierter Alkylrest, die zusammen 7 bis 15 Kohlenstoffatomen aufweisen, verstanden, wobei der Arylrest weitere Substituenten, wie beispielsweise ein Halogenatom tragen kann. Beispiele sind eine freie oder aromatisch substituierte Benzylgruppe, wie eine Benzylgruppe oder eine Halogenbenzylgruppe.

Unter dem Begriff "C₇₋₁₅-Aralkyloxygruppe" wird im Sinne der vorliegenden Anmeldung eine um ein Sauerstoffatom verlängerte "C₇₋₁₅-Aralkylgruppe", wie beispielsweise eine Benzyloxygruppe, verstanden.

Unter dem Begriff "Halogen" wird im Rahmen der vorliegenden Erfindung ein Fluor-, Chlor-, Brom- oder lodatom verstanden.

Unter dem Begriff "Pseudohalogen" wird im Sinne der vorliegenden Anmeldung eine Cyanat-, Rhodanid-, Cyan- oder Azidgruppe verstanden.

Unter dem Begriff "Perfluoralkylrest" wird im Sinne der vorliegenden Anmeldung ein verzweigter oder geradkettiger Fluoralkylrest mit 1 bis 3 Kohlenstoffatomen verstanden, wobei Beispiele eine Trifluormethyl-, Pentafluorethyl-, Heptafluor-n-propyl- oder Heptafluor-iso-propylgruppe sind.

R₁ bedeutet vorzugsweise eine Hydroxy- oder C₁₋₁₁-Acyloxygruppe, insbesondere eine Formyloxygruppe, Acetyloxygruppe, Propionyloxygruppe, n-Butyryloxygruppe, iso-Butyryloxygruppe, Heptanyloxygruppe oder Undecanyloxygruppe.

Bedeutet R₂ einen Rest -(CH₂)ₙCH₂Y ist n vorzugsweise gleich 1 und Y steht vorzugsweise für ein Fluoratom, eine Cyan- oder Rhodanidgruppe. Bedeutet R₂ einen Rest -(CH₂)ₘ-CH=CH(CH₂)ₚ-R₆ ist m vorzugsweise gleich 1 und R₆ steht vorzugsweise für eine Methyl- oder Ethylgruppe oder eine Methoxy- oder Ethoxygruppe.

Bedeutet R₂ einen Rest -(CH₂)ₒC≡CR₇ ist o vorzugsweise gleich 1 und R₇ steht vorzugsweise für ein Fluoratom oder für eine Methyl- oder Ethylgruppe.

Besonders bevorzugt bedeutet R₂ ein Wasserstoffatom oder einen C₁₋₆Alkylrest, insbesondere eine Methyl- oder Ethylgruppe.

R₃ bedeutet vorzugsweise einen C₁₋₄-Alkylrest, besonders bevorzugt eine Methylgruppe.

R₄ bedeutet vorzugsweise ein Fluor-, Chlor- oder Bromatom oder eine Trifluormethyloder Hydroxygruppe. R₅ bedeutet vorzugsweise eine Methyl- oder Ethylgruppe.

Am stärksten bevorzugt sind die folgenden Verbindungen:
1) 4-Chlor-17β-Hydroxy-14α,15α-methylen-estr-4-en-3-on,
2) 4-Chlor-17α-Hydroxy-14α,15α-methylen-estr-4-en-3-on,
3) 4-Chlor-17β-Hydroxy-14β,15β-methylen-estr-4-en-3-on,
4) 4-Chlor-17α-Hydroxy-14β,15β-methylen-estr-4-en-3-on,
5) 4-Brom-17β-Hydroxy-14α,15α-methylen-estr-4-en-3-on,
6) 4-Brom-17α-Hydroxy-14α,15α-methylen-estr-4-en-3-on,
7) 4-Brom-17β-Hydroxy-14β,15β-methylen-estr-4-en-3-on,
8) 4-Brom-17α-Hydroxy-14β,15β-methylen-estr-4-en-3-on,
9) 4-Fluor-17β-Hydroxy-14α,15α-methylen-estr-4-en-3-on,
10) 4-Fluor-17α-Hydroxy-14α,15α-methylen-estr-4-en-3-on,
11) 4-Fluor-17β-Hydroxy-14β,15β-methylen-estr-4-en-3-on,
12) 4-Fluor-17α-Hydroxy-14β,15β-methylen-estr-4-en-3-on,
13) 4,17β-Dihydroxy-14α,15α-methylen-estr-4-en-3-on,
14) 4,17α-Dihydroxy-14α,15α-methylen-estr-4-en-3-on,
15) 4,17β-Dihydroxy-14β,15β-methylen-estr-4-en-3-on,
16) 4,17α-Dihydroxy-14β,15β-methylen-estr-4-en-3-on,
17) 4-Trifluormethyl-17β-Hydroxy-14α,15α-methylen-estr-4-en-3-on,
18) 4-Trifluormethyl-17α-Hydroxy-14α,15α-methylen-estr-4-en-3-on,
19) 4-Trifluormethyl-17β-Hydroxy-14β,15β-methylen-estr-4-en-3-on,
20) 4-Trifluormethyl-17α-Hydroxy-14β,15β-methylen-estr-4-en-3-on,
22) 17β-Hydroxy-14α,15α-methylen-estra-4,9,11-trien-3-on,
23) 17α-Hydroxy-14α,15α-methylen-estra-4,9,11-trien-3-on,
24) 17β-Hydroxy-14α,15α-methylen-estra-4,9,11-trien-3-on,
25) 17β-Hydroxy-14β,15β-methylen-estra-4,9,11-trien-3-on und
26) 17α-Hydroxy-14β,15β-methylen-estra-4,9,11-trien-3-on.

Die erfindungsgemäßen Verbindungen der Formel (I) können hergestellt werden, indem man in 14,15-Cyclopropanosteroide der allgemeinen Formel (II) 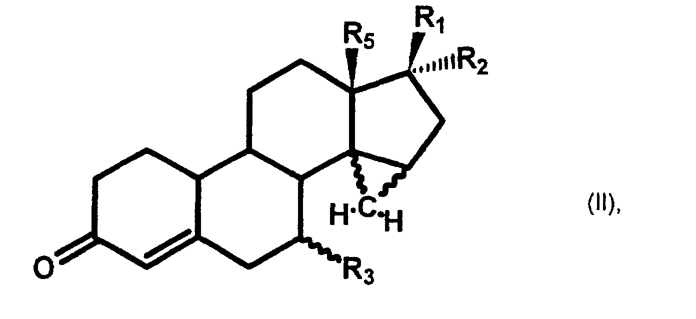 worin R₁, R₂, R₃ und R₅ die vorstehend gegebene Bedeutung besitzen, die 4,5-Doppelbindung mit Wasserstoffperoxid unter alkalischen Bedingungen epoxidiert und das entstandene Epoxidgemisch in einem geeignetem Lösungsmittel mit Säuren der allgemeinen Formel HR₈ behandelt, wobei R₈ ein Halogenatom oder ein Pseudohalogen sein kann, oder mit katalytischen Mengen Mineralsäure umsetzt und gegebenenfalls die vorstehend erhaltenen 4-Bromverbindungen der allgemeinen Formel (I) mit 2,2-Difluor-2-(fluorsulfonyl)essigsäuremethylester in Dimethylformamid in Gegenwart von CuJ umsetzt.

Die Herstellung der Verbindungen der Formel (II) erfolgt gemäß bekannter Verfahren bzw. ist in der deutschen Anmeldung Nr. 198 27 522.6 (PCT/DE99/01795) beschrieben. In dem genannten Schutzrecht ist auch die Einführung der zu den hier beanspruchten Resten R₁, R₂, R₃ und R₅ dort analog vorkommenden Resten beschrieben.

Des weiteren können entsprechende 4-Bromverbindungen auch durch Addition von Brom mittels Brom, N-Bromsuccinimid oder N-Bromacetamid an Verbindungen der allgemeinen Formel (II) in Essigsäure/Ether in Gegenwart eines Protonenakzeptors, wie zum Beispiel Kollidin, hergestellt werden (X. S. Fei et. al., J. Chem. Soc. Perkin Trans. 1, 1998, 1139-1142).

4-Trifluormethylverbindungen der allgemeinen Formel (I) können durch Umsetzung der voranstehend erhaltenen 4-Bromverbindungen der allgemeinen Formel (I) mit 2,2-Difluor-2-(fluorsulfonyl)-essigsäuremethylester in Dimethylformamid in Gegenwart von CuJ erhalten werden (X. S. Fei et. al., J.Chem. Soc., Perkin Trans. 1, 1998, 1139-1142).

4-Hydroxyverbindungen werden durch Umsetzung des oben genannten Epoxidgemisches mit katalytischen Mengen Mineralsäure, wie zum Beispiel Schwefelsäure, erhalten (P. S. Furth et. al., J. Enzyme Inhibition, 1990, Bd. 4, 131-135).

Verbindungen der allgemeinen Formel (I), welche eine 4,9,11-Trienstruktur enthalten, können aus Verbindungen der allgemeinen Formal (III) 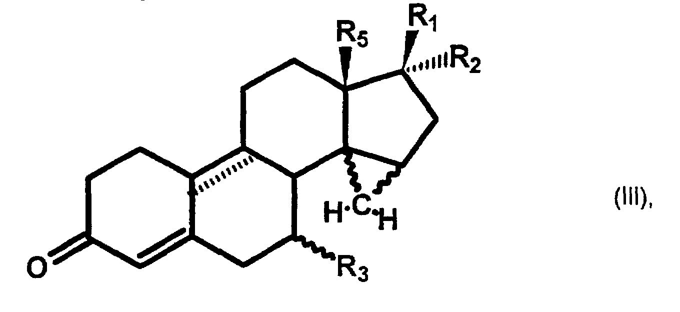 worin R₁, R₂, R₃ und R₅ die vorstehend genannte Bedeutung besitzen (hergestellt beispielsweise nach EP 0 768 316 A1) nach aus der Literatur bekannten Methoden in die Verbindungen mit 4,9,11-Trien-Struktur der allgemeinen Formel (I) überführt werden.

So kann man z. B. Verbindungen der allgemeinen Formel (III) in 3-Stellung ketalisieren, wobei ein 5(10),9(11)-Dien entsteht. Nach vorsichtiger Hydrolyse der Ketalgruppe wird dann ein 5(10),9(11)-Dien-3-on erhalten, welches sich leicht mit Dichlordicyanobenzochinon zu den gewünschten Verbindungen der allgemeinen Formel (I) dehydrieren läßt (nach M. Heller, R. H. Lenhard, S. Bernstein, Steroids 1967, S. 211-217). Außerdem ist es möglich das voranstehend genannte 5(10),9(11)-Dien-3-on in das 11β-Hydroperoxid zu überführen. Nach Reduktion des 11β-Hydroperoxids zur 11β-Hydroxyverbindung kann die Hydroxygruppe sauer, unter Bildung der gewünschten Verbindungen der allgemeinen Formel (I) abgespalten werden. (Nach L. Nedelec, V. Torelli, G. Costerousse, V. Delaroff, Bull. Soc. Chim., 1977, S. 670-675).

Gegenstand der vorliegenden Erfindung sind auch Arzneimittel zur oralen, rektalen, subcutanen, intravenösen oder intramuskulären Anwendung, die zusammen mit den üblichen Trägem und Verdünnungsmitteln mindestens eine Verbindung der allgemeinen Formel (I) und/oder deren Säureadditionssalze als Wirkstoff enthalten.

Pharmazeutische Präparate der Erfindung werden mit den üblichen festen oder flüssigen Trägerstoffen und/oder Verdünnungsmitteln und den allgemein üblicherweise verwendeten Hilfsstoffen entsprechend der gewünschten Applikationsart in einer geeigneten Dosierung und in an sich bekannter Weise hergestellt. Bei einer bevorzugten oralen Darreichungsform werden vorzugsweise Tabletten, Filmtabletten, Dragees, Kapseln, Pillen, Pulver, Lösungen oder Suspensionen auch als Depotform zubereitet.

Daneben sind parenterale Arzneiformen wie Injektionslösungen oder aber Suppositorien in Betracht zu ziehen.

Arzneiformen als Tabletten können beispielsweise durch Mischen des Wirkstoffes mit bekannten Hilfsstoffen, wie Dextrose. Zucker, Sorbit, Mannit, Polyvinylpyrrolidon, Sprengmitteln, wie Maisstärke oder Alginsäure, Bindemitteln, wie Stärke oder Gelatine, Gleitmitteln, wie Magnesiumstearat, oder Talk und/oder Mitteln, die einen Depoteffekt erzielen können, wie Carboxylpolymethylen, Carboxymethylcellulose, Celluloseacetatphthalat oder Polyvinylacetat, erhalten werden. Die Tabletten können auch aus mehreren Schichten bestehen.

Analog lassen sich Dragees durch Überziehen von analog den Tabletten hergestellten Kernen mit üblicherweise in Drageeüberzügen verwendeten Mitteln, beispielsweise

Polyvinylpyrrolidon oder Schellack, Gummiarabicum, Talk, Titandioxid, oder Zucker bereiten. Die Drageehülle kann dabei auch aus mehreren Schichten bestehen, wobei beispielsweise die oben genannte Hilfsstoffe verwendet werden.

Die Lösungen oder Suspensionen mit dem erfindungsgemäßen Wirkstoff können zur Verbesserung des Geschmacks mit Stoffen, wie Saccharin, Cyclamat oder Zucker und/oder mit Aromastoffen, wie Vanillin oder Orangenextrakt, versetzt werden. Weiterhin können sie mit Suspendierhilfsstoffen, wie Natriumcarboxymethylcellulose, oder Konservierungsmitteln, wie p-Hydroxybenzoesäure, vermischt werden.

Die Bereitung von Kapseln kann durch Mischen des Arzneistoffes mit Trägern, wie Milchzucker oder Sorbit, erfolgen, die dann in die Kapseln eingebracht werden.

Die Herstellung von Suppositorien erfolgt vorzugsweise durch Mischung des Wirkstoffes mit geeigneten Trägermaterialien, wie Neutralfetten oder Polyethylenglykolen, oder deren Derivaten.

Bei den pharmazeutischen Zubereitungsformen kann es sich weiterhin um perkutane Zubereitungsformen, z. B. transdermale therapeutische Systeme (TTS) oder Gele, Sprays oder Salben oder um intranasale Zubereitungsformen, wie Nasenspray oder Nasentropfen, handeln.

Die erfindungsgemäßen 14,15-Cyclopropanosteroide der 19-Norandrostan-Reihe der allgemeinen Formel (I) sind hormonell (gestagen und/oder androgen) wirkende Verbindungen.

So bindet zum Beispiel 4-Chlor-17β-Hydroxy-14α,15α-methylen-estr-4-en-3-on mit 98% an den Androgenrezeptor (R 1881= 100%) und mit 5,5% an den Progesteronrezeptor (Progesteron = 100%). Dagegen bindet 17β-Hydroxy-14α,15α-methylen-estra-4,9,11-trien-3-on mit 58% an den Androgenrezeptor und mit 36 % an den Progesteronrezeptor.

Diese Testergebnisse eröffnen den erfindungsgemäßen Verbindungen der allgemeinen Formel (I) vielfältige Möglichkeiten für die Fertilitätskontrolle bei Mann und Frau, die Hormonreplacement-Therapie bei Mann und Frau oder die Behandlung hormonell bedingter Erkrankungen bei Mann und Frau, wie beispielsweise Endometriose, Mammacarcinom oder Hypogonadismus.

Die nachfolgenden Beispielen dienen der näheren Erläuterung der vorliegenden Erfindung und sollen diese nicht einschränken.

### Beispiele

### Beispiel 1

### 4-substituierte Verbindungen aus 4,5-Epoxiden

### Stufe 1: Herstellung von 4,5 Epoxiden

### 4ξ,5ξ-Epoxy-17β-Hydroxy-14α,15α-methylen-estran-3-on

2g 17β-Hydroxy-14α,15α-methylen-estr-4-en-3-on werden in 50 ml Methanol gelöst und bei 10 °C mit 20 ml Wasserstoffperoxidlösung (35%) versetzt. Unter Rühren werden 5 ml 10 %-ige Natriumhydroxidlösung zugegeben und es wird 3 Stunden gerührt. Die Reaktionslösung wird mit 50 ml Dichlormethan und 25 ml Wasser versetzt und die organische Phase wird abgetrennt. Man wäscht mit halbkonzentrierter Thiosulfatlösung, trocknet und engt bis zur Trockene ein. Der erhaltene Rückstand besteht aus einem Gemisch von 4α,5α- bzw. 4β,5β-Epoxiden und wird ohne weitere Reinigung in der Folgestufe eingesetzt.

### Stufe 2: 4-substituierte Verbindungen

### 4-Chlor-17β-Hydroxy-14α,15α-methylenestr-4-en-3-on

2 g Epoxidgemisch (Stufe 1) werden in 200 ml Aceton gelöst und bei 5°C mit 12 ml konzentrierter Salzsäure versetzt. Nach 2 Stunden wird mit Sodalösung neutralisiert und das Aceton wird abgezogen. Der Rückstand wird mit Dichlormethan ausextrahiert. Die organischen Extrakte werden getrocknet und eingeengt. Nach Kristallisation aus Ethanol erhält man 4-Chlor-17β-Hydroxy-14α,15α-methylenestr-4-en-3-on.
¹H-NMR: 0,19 (2H, m, CH₂-Brücke), 1,02 (3H, s, H-18), 3,50 (1H, dd, J=9,4, 6,6 Hz, H-17).

### Beispiel 2

### 4-Brom-17β-hydroxy-14α,15α-methylenestr-4-en-3-on

In zu 4-Chlor-17β-Hydroxy-14α,15α-methylenestr-4-en-3-on analoger Weise erhält man 4-Brom-17β-hydroxy-14α,15α-methylenestr-4-en-3-on, wenn man anstatt Salzsäure 48%-ige Bromwasserstoffsäure verwendet.
¹H-NMR: 0,22 (2H, m , CH₂-Brücke), 1,08 (3H, s, H-18), 3,48 (1H, dd, H-17).

### Beispiel 3

### 4,17β-Dihydroxy-14α,15α-methylenestr-4-en-3-on

2 g Epoxidgemisch (Stufe 1) werden in 20 ml Essigsäure, welche 2 Vol-% konzentrierte Schwefelsäure enthält, gelöst. Die Lösung wird 3 Tage bei 10 °C stehengelassen. Danach versetzt man mit 200 ml Essigsäureethylester und neutralisiert mit Sodalösung. Die organische Phase wird getrocknet und eingeengt. Der Rückstand wird in 50 ml Methanol gelöst, mit 2 g Kaliumhydroxid versetzt und 1 Stunde am Rückfluß erhitzt. Man läßt abkühlen, neutralisiert mit 50 %-iger Essigsäure, gießt in 500 ml Wasser und saugt die Kristalle ab. Man erhält 4,17β-Dihydroxy-14α,15α-methylenestr-4-en-3-on.
¹H-NMR: 0,20 (2H, m, CH₂-Brücke), 1,10 (3H, s, H-18), 3,50 (1H, m, H-17), 6.10 (1H, s, 4-OH).

### Beispiel 4

### 4-Trifluormethyl-17β-hydroxy-14α,15α-methylenestr-4-en-3-on

1g 4-Brom-17β-hydroxy-14α,15α-methylenestr-4-en-3-on wird in 100 ml Dimethylformamid gelöst und mit 0,5 g Cul sowie 2 ml 2,2-Difluor-2-(fluorsulfonyl)-essigsäuremethylester bei 80 °C 15 Stunden gerührt. Nach Aufarbeitung und chromatografischer Reinigung erhält man 4-Trifluormethyl-17β-Hydroxy-14α,15α-methylenestr-4-en-3-on.
¹H-NMR: 0,22 (2H, m, CH₂-Brücke), 1,06 (3H, s, H-18), 3,50 (1H, dd, J=9,4, 6,6 Hz, H-17).
¹⁹F-NMR: -57,2 (3F, s , 4-F₃C).

### Beispiel 5

### Herstellung von 4,9,11-Trienen

### 17β-Hydroxy-14α,15α-methylanestr-4,9,11-trien-3-on

4g 17β-Hydroxy-14α,15α-methylenestr-4,9,-dien-3-on werden in 40 ml Methanol gelöst und mit 16 ml Trimethylorthoformiat sowie 50 mg p-Toluolsulfonsäure versetzt. Es wird 1,5 Stunden bei 25 °C gerührt und dann wird mit 1N methanolischer Kaliumhydroxidlösung auf pH 9 eingestellt, es werden 50 ml Wasser zugegeben und das Methanol wird im Vakuum abdestilliert. Die ausgefallene Substanz wird abgesaugt und mit Wasser gewaschen. Das erhaltene 3,3-Dimethoxy-14α,15α-methylenestr-5(10),9,11,-dien-17β-ol ( 4,3 g) wird in 50 ml 80%-igem wäßrigen Aceton gelöst und es werden 1,6 ml 20 %-ige Schwefelsäure zugetropft. Nach 1,5 Stunden Rühren bei Raumtemperatur werden 2,5 ml Triethylamin und 200 ml Wasser zugegeben, wobei das Produkt fest ausfällt. Es wird abgesaugt und mit Wasser gewaschen. Man erhält 17β-Hydroxy-14α,15α-methylenestr-5(10)-9(11)-dien-3-on (3,8 g). Das erhaltene Produkt (3,8 g) wird in 80 ml Dioxan mit 6 g Dichlordicyanobenzochinon 3,5 Stunden bei 40 °C gerührt. Man läßt abkühlen, saugt den Niederschlag ab, wäscht mit Dioxan und engt die Filtrate zur Trockene ein. Der Rückstand wird an Kieselgel chrorhatographiert, wobei man 17β-Hydroxy-14α,15α-methylenestr-4,9,11-trien-3-on erhält,
¹H-NMR: 0,18 (1H, m, CH₂-Brücke), 0,27 (1H, m, CH₂-Brücke), 1,15 (3H, s, H-18), 3,77 (1H, dd, J=9,0, 7,0 Hz, H-17), 6,42 und 6,51 (2H, 2 d, J=9,6 Hz, H-11,H-12).

## Patentansprüche

1. 14,15-Cyclopropanosteroide der 19-Norandrostan-Reihe der allgemeinen Formel (I) 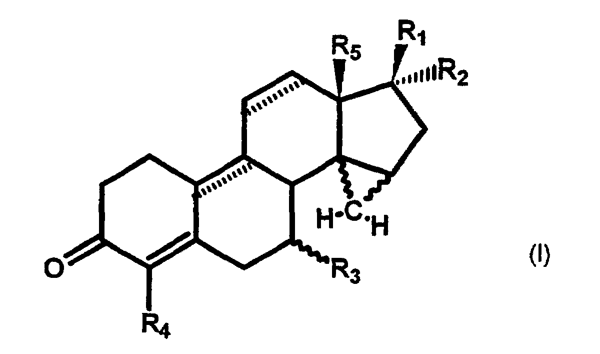
worin R₁ ein Wasserstoffatom, eine Hydroxygruppe, eine C₁₋₁₀-Alkyl-, C₁₋₁₀-Alkytoxy-, C₁₋₁₅-Acyloxy-, C₄₋₁₅-Aryloxy-, C₇₋₁₅-Aralkyloxy- oder eine C₇₋₁₅-Alkylaryloxygruppe ist,
R₂ für ein Wasserstoffatom, eine Hydroxygruppe, eine C₁₋₁₀-Alkyl-, C₁₋₁₀-Acyl-, C₁₋₁₀-Acyloxy-, C₆₋₁₅-Aryl-, C₇₋₁₅-Aralkyl- oder C₇₋₁₅-Alkylarylgruppe,
für einen Rest -(CH₂)ₙCH₂Y
mit n = 0, 1 oder 2 und Y für ein Halogenatom, insbesondere ein Fluor-, Chlor-, Brom- oder Jodatom, für ein Pseudohalogen, insbesondere eine Cyan-, Azid- oder Rhodanidgruppe,
für einen Rest - (CH₂)m-CH=CH(CH₂)p-R₆
mit m = 0, 1, 2 oder 3 und p = 0, 1 oder 2 und R₆ für ein Wasserstoffatom, einen C₁₋₁₀-Alkyl-, C₆₋₁₅-Aryl-, C₇₋₁₅-Aralkyl- oder C₇₋₁₅-Alkylarylrest oder eine Hydroxylgruppe, eine C₁₋₁₀-Alkyloxygruppe oder eine C₁₋₁₀-Acyloxygruppe,
für einen Rest -(CH₂)oC≡CR₇
mit o = 0, 1 oder 2 und R₇ für ein Wasserstoffatom, ein Halogenatom, insbesondere ein Fluor-, Chlor-, Brom- oder lodatom, einen C₁₋₁₀-Alkyl-, C₆₋₁₅-Aryl-, C₇₋₁₅-Aralkyl-, C₇₋₁₅-Alkylaryl- oder C₁₋₁₀-Acylrest steht,
R₁ und R₂ zusammen für eine Keto-, Methylen-, Difluormethylengruppe stehen oder unter Einschluß des C-17 ein Spirooxiran oder ein 2,2-Dimethyl-1,3-dioxolan bilden,
R₃ für ein Wasserstoffatom oder eine α- oder β-ständige C₁₋₁₀-Alkylgruppe steht,
R₄ für ein Halogenatom, insbesondere ein Fluor-, Chlor- oder Bromatom, oder ein Pseudohalogen, insbesondere eine Rhodanid- oder Azidgruppe, oder eine Hydroxyl- oder Perfluoralkylgruppe steht und
R₅ für eine C₁₋₄-Alkylgruppe steht, sich zwischen C-14 und C-15 eine α- oder β-Cyclopropangruppe befindet,
sich in 9,10- oder 11,12-Stellung Doppelbindungen befinden können,
mit der Maßgabe, daß, wenn sich in 9,10- und 11,12-Stellung zwei weitere Doppelbindungen befinden oder sich in 11,12-Stellung eine Doppelbindung
befindet, R₄ zusätzlich zu den voranstehend genannten Bedeutungen ein Wasserstoffatom sein kann, und mit der weiteren Maßgabe, daß, wenn R₅ keine Methylgruppe ist, R₄ zusätzlich zu den voranstehend genannten Bedeutungen ein Wasserstoffatom sein kann,
sowie deren pharmazeutisch verträgliche Salze.

2. Verbindungen nach Anspruch 1, wobei R₁ eine Hydroxy- oder Acyloxyguppe, insbesondere eine Formyloxygruppe, Acetyloxygruppe, Propionyloxygruppe, Butyryloxygruppe, iso-Butyryloxygruppe, Heptanyloxygruppe oder Undecanyloxygruppe, darstellt.

3. Verbindungen nach Anspruch 1 oder 2, wobei R₂ ein Wasserstoffatom oder eine Alkylgruppe, insbesondere ein Methyl- oder Ethylgruppe, darstellt.

4. Verbindungen nach einem der Ansprüche 1 bis 3, wobei R₃ eine Methylgruppe ist.

5. Verbindungen nach einem der Ansprüche 1 bis 4, wobei R₄ ein Fluor-, Chlor- oder Bromatom oder eine Trifluormethyl- oder Hydroxygruppe darstellt.

6. Verbindungen nach einem der Ansprüche 1 bis 5, wobei R₅ eine Methyl- oder Ethylgruppe darstellt.

7. Verbindungen nach Anspruch 1, nämlich
1) 4-Chlor-17β-Hydroxy-14α,15α-methylen-estr-4-en-3-on,
2) 4-Chlor-17α-Hydroxy-14α,15α-methylen-estr-4-en-3-on,
3) 4-Chlor-17β-Hydroxy-14β,15β-methylen-estr-4-en-3-on,
4) 4-Chlor-17α-Hydroxy-14β,15β-methylen-estr-4-en-3-on,
5) 4-Brom-17β-Hydroxy-14α,15α-methylen-estr-4-en-3-on,
6) 4-Brom-17α-Hydroxy-14α,15α-methylen-estr-4-en-3-on,
7) 4-Brom-17β-Hydroxy-14β,15β-methylen-estr-4-en-3-on,
8) 4-Brom-17α-Hydroxy-14β,15β-methylen-estr-4-en-3-on,
9) 4-Fluor-17β-Hydroxy-14α,15α-methylen-estr-4-en-3-on,
10) 4-Fluor-17α-Hydroxy-14α,15α-methylen-estr-4-en-3-on,
11) 4-Fluor-17β-Hydroxy-14β,15β-methylen-estr-4-en-3-on,
12) 4-Fluor-17α-Hydroxy-14β,15β-methylen-estr-4-en-3-on,
13) 4,17β-Dihydroxy-14α,15α-methylen-estr-4-en-3-on,
14) 4,17α-Dihydroxy-14α,15α-methylen-estr-4-en-3-on,
15) 4,17β-Dihydroxy-14β,15β-methylen-estr-4-en-3-on,
16) 4,17α-Dihydroxy-14β,15β-methylen-estr-4-en-3-on,
17) 4-Trifluormethyl-17β-Hydroxy-14α,15α-methylen-estr-4-en-3-on,
18) 4-Trifluormethyl-17α-Hydroxy-14α,15α-methylen-estr-4-en-3-on,
19) 4-Trifluormethyl-17β-Hydroxy-14β,15β-methylen-estr-4-en-3-on,
20) 4-Trifluormethyl-17α-Hydroxy-14β,15β-methylen-estr-4-en-3-on,
22) 17β-Hydroxy-14α,15α-methylen-estra-4,9,11-trien-3-on,
23) 17α-Hydroxy-14α,15α-methylen-estra-4,9,11-trien-3-on,
24) 17β-Hydroxy-14α,15α-methylen-estra-4,9,11 -trien-3-on,
25) 17β-Hydroxy-14β,15β-methylen-estra-4,9,11-trien-3-on und
26) 17α-Hydroxy-14β,15β-methylen-estra-4,9,11-frien-3-on.

8. Verfahren zur Herstellung der Verbindungen nach einem der Ansprüche 1 bis 7 **dadurch gekennzeichnet, daß** man in 14,15-Cyclopropanosteroide der allgemeinen Formel (II) 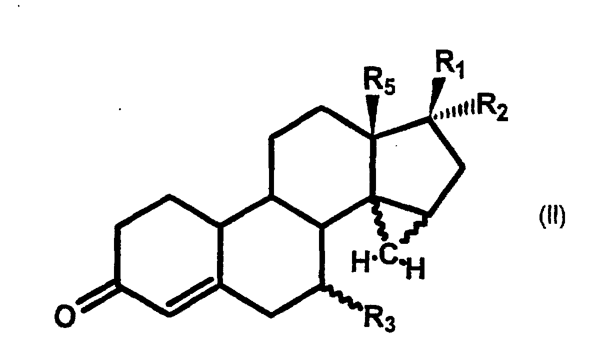 worin R₁, R₂, R₃ und R₅ die in den Ansprüchen 1 bis 7 genannte Bedeutung besitzen, die 4,5-Doppelbindung mit Wasserstoffperoxid unter alkalischen Bedingungen epoxidiert und das entstandene Epoxidgemisch in einem geeignetem Lösungsmittel mit Säuren der allgemeinen Formel HR₈ behandelt, wobei R₈ ein Halogenatom, insbesondere ein Fluor-, Chlor- oder Bromatom, ein Pseudohalogen, insbesondere eine Azid- oder Rhodanogruppe, sein kann, oder mit katalytischen Mengen Mineralsäure umsetzt und gegebenenfalls die vorstehend erhaltenen 4-Bromverbindungen der allgemeinen Formel (I) mit 2,2-Difluor-2-(fluorsulfonyl)essigsäuremethylester in Dimethylformamid in Gegenwart von CuJ umsetzt.

9. Verfahren zur Herstellung der Verbindungen nach einem der Ansprüche 1 bis 7, **dadurch gekennzeichnet, daß** man Verbindungen der allgemeinen Formel (III) 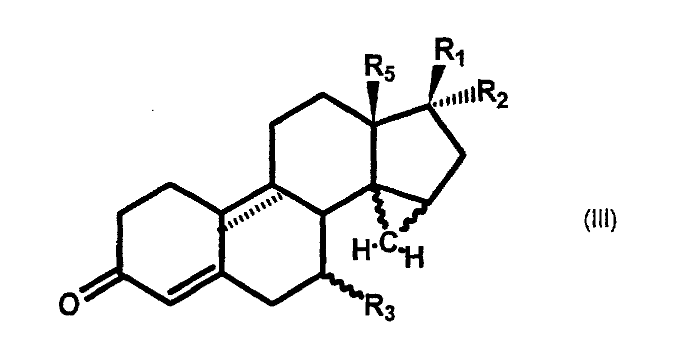 worin R₁, R₂, R₃ und R₅ die in den Ansprüchen 1 bis 7 genannte Bedeutung besitzen, in an sich bekannter Weise durch Ketalisierung der Verbindungen der allgemeinen Formel (III), Hydrolyse der Ketalgruppe des dabei entstandenen 5(10),9(11)-Diens und Umsetzung des erhaltenen 5(10),9(11)-Dien-3-ons mit Dichlordicyanobenzochinon in die gewünschten Verbindungen der allgemeinen Formel (I) mit 4,9,11-Trien-Struktur überführt.

10. Pharmazeutische Zusammensetzung, die mindestens eine Verbindung der allgemeinen Formel (I) nach den Ansprüchen 1 bis 7, gegebenenfalls zusammen mit pharmazeutisch verträglichen Hilfs- und Trägerstoffen enthält.

11. Verwendung der Verbindungen der allgemeinen Formel (I) nach den Ansprüchen 1 bis 7 zur Herstellung eines Medikaments zur Fertilitätskontrolle beim Mann, der Hormon-Replacement-Therapie (HRT) bei Mann und Frau oder der Behandlung hormonell bedingter Erkrankungen bei Mann und Frau, wie beispielsweise Endometriose, Mammacarcinom oder Hypogonadismus.

12. Verbindungen der allgemeinen Formel (I) nach den Ansprüchen 1 bis 7 zur Anwendung als therapeutische Wirkstoffe.

## Claims

1. 14,15-Cyclopranoandrostanes of the 19-norandrostane series of the general formula (I) 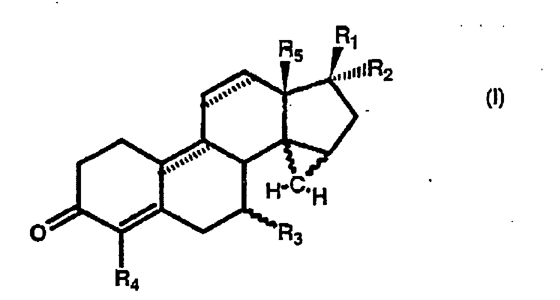
in which R₁ is a hydrogen atom, a hydroxy group, C₁₋₁₀-alkyl, C₁₋₁₀-alkyloxy, C₁₋₁₅ acyloxy, C₄₋₁₅-aryloxy, C₇₋₁₅-aralkyloxy or a C₇₋₁₅-alkylaryloxy group
R₂ represents a hydrogen atom, a hydroxy group, a C₁₋₁₀ alkyl, C₁₋₁₀ acyl, C₁₋₁₀ acyloxy, C₆₋₁₅ aryl, C₇₋₁₅ aralkyl or C₇₋₁₅ alkylaryl group
a -(CH₂)ₙCH₂Y group
with n = 0, 1 or 2 and Y represents a halogen atom, especially a fluorine, chlorine, bromine or iodine atom, a pseudohalogen, especially a cyano, azide or rhodanide group, -(CH₂)ₘ-CH=CH(CH₂)ₚ-R₆ group
with m = 0, 1, 2 or 3 and p = 0, 1 or 2 and R₆ represents a hydrogen atom, a C₁₋₁₀ alkyl, C₆₋₁₅ aryl, C₇₋₁₅ aralkyl or C₇₋₁₅ alkylaryl group or a hydroxyl group, a C₁₋₁₀ alkyloxy group or a C₁₋₁₀ acyloxy group,
a -(CH₂)ₒC□CR₇ group
with o = 0, 1 or 2 and R₇ represents a hydrogen atom, a halogen atom, especially a fluorine, chlorine, bromine or iodine atom, a C₁₋₁₀ alkyl, C₆₋₁₅ aryl, C₇₋₁₅ aralkyl, C₇₋₁₅ alkylaryl or C₁₋₁₀ acyl group
R₁ and R₂ together represent a keto, methylene, difluoromethylene group or, with inclusion of the C-17, a spirooxirane or a 2,2-dimethyl-1,3-dioxolane,
R₃ represents a hydrogen atom or an α or β C₁₋₁₀ alkyl group,
R₄ represents a halogen atom, especially a fluorine, chlorine or bromine atom, or a pseudohalogen, especially a rhodanide or azide group, or a hydroxyl or perfluoroalkyl group and
R₅ represents a C₁₋₄ alkyl group, there being an α- or β-cyclopropane group between C-14 and C-15,
and possibly double bonds in the 9,10 or 11,12 position,
with the proviso that, if there are 2 further double bonds in the 9,10 and 11,12 positions or one double bond in the 11,12 position, R₄ can be a hydrogen atom in addition to the meanings given above and with the further proviso that, when R₅ is a methyl group, R₄ can be a hydrogen atom in addition to the meanings given above,
as well as their pharmaceutically tolerated salts.

2. Compounds of claim 1, in which R₁ represents a hydroxy or acyloxy group, especially a formyloxy, acetyloxy, propionyloxy, n-butyryloxy, iso-butyryloxy, heptanyloxy or undecanyloxy group.

3. Compounds of claims 1 or 2, in which R₂ represents a hydrogen atom or an alkyl group, especially a methyl or ethyl group.

4. Compounds of one of the claims 1 to 3, in which R₃ represents a methyl group.

5. Compounds of one of the claims 1 to 4, in which R₄ represents a fluorine, chlorine or bromine atom or a trifluoromethyl or hydroxy group.

6. Compounds of one of the claims 1 to 5, in which R₅ represents a methyl or ethical group.

7. Compounds of claim 1, namely
1) 4-chloro-17β-hydroxy-14α,15α-methylene-estr-4-ene-3-one
2) 4-chloro-17α-hydroxy-14α,15α-methylene-estr-4-ene-3-one
3) 4-chloro-17β-hydroxy-14β,15β-methylene-estr-4-ene-3-one
4) 4-chloro-17α-hydroxy-14β,15β-methylene-estr-4-ene-3-one
5) 4-bromo-17β-hydroxy-14α,15α-methylene-estr-4-ene-3-one
6) 4-bromo-17α-hydroxy-14α,15α-methylene-estr-4-ene-3-one
7) 4-bromo-17β-hydroxy-14β,15β-methylene-estr-4-ene-3-one
8) 4-bromo-17α-hydroxy-14β,15β-methylene-estr-4-ene-3-one
9) 4-fluoro-17β-hydroxy-14α,15α-methylene-estr-4-ene-3-one
10) 4-fluoro-17α-hydroxy-14α,15α-methylene-estr-4-ene-3-one
11) 4-fluoro-17β-hydroxy-14β,15β-methylene-estr-4-ene-3-one
12) 4-fluoro-17α-hydroxy-14β,15β-methylene-estr-4-ene-3-one
13) 4,17β-dihydroxy-14α,15α-methylene-estr-4-ene-3-one
14) 4,17α-dihydroxy-14α,15α-methylene-estr-4-ene-3-one
15) 4,17β-dihydroxy-14β,15β-methylene-estr-4-ene-3-one
16) 4,17α-dihydroxy-14β,15β-methylene-estr-4-ene-3-one
17) 4-trifluoromethyl-17β-hydroxy-14α,15α-methylene-estr-4-ene-3-one
18) 4-trifluoromethyl-17α-hydroxy-14α,15α-methylene-estr-4-ene-3-one
19) 4-trifluoromethyl-17β-hydroxy-14β,15β-methylene-estr-4-ene-3-one
20) 4-trifluoromethyl-17α-hydroxy-14β,15β-methylene-estr-4-ene-3-one
22) 17β-hydroxy-14α,15α-methylene-estra-4,9,11-triene-3-one
23) 17α-hydroxy-14α,15α-methylene-estra-4,9,11-triene-3-one
24) 17β-hydroxy-14α,15α-methylene-estra-4,9,11-triene-3-one
25) 17β-hydroxy-14β,15β-methylene-estra-4,9,11-triene-3-one and
26) 17α-hydroxy-14β,15β-methylene-estra-4,9,11-triene-3-one

8. A method for the synthesis of compounds of one of the claims 1 to 7, wherein, in 14, 15-cyclopropanosteriods of the general formula (II) 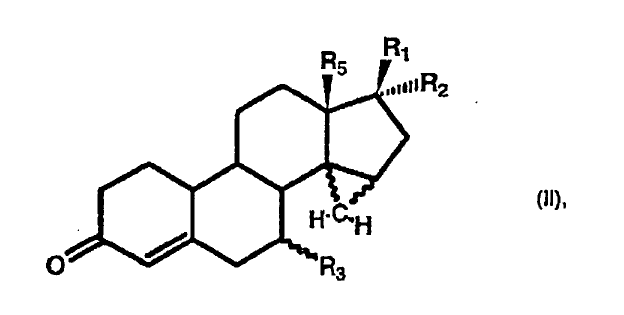 in which R₁, R₂, R₃, R₅ have the meaning given in claims 1 to 7, the 4, 5 double bond is epoxidized with hydrogen peroxide under alkaline conditions and the resulting epoxide mixture is treated in a suitable solvent with acids having the general formula HR₈, R₈ being a halogen atom, especially a fluorine, chlorine or bromine atom, a pseudohalogen atom, especially an azide or a rhodanide group or reacted with catalytic amounts of a mineral acid and the 4-bromo compound of the general formula (I), obtained above, is reacted with methyl 2,2-difluoro-2-(fluorosulfonyl) acetate in dimethylformamide in the presence of CuI.

9. A method for the synthesis of compounds of one of the claims 1 to 7, wherein compounds of the general formula (III) 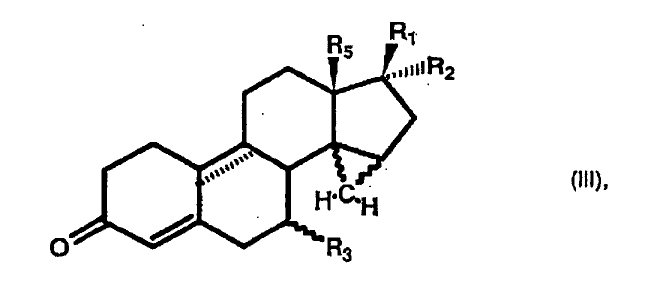 in which R₁, R₂, R₃ and R₅ have the meaning given in claims 1 to 7, are converted into the desired compounds of the general formula (I) with a 4,9,11-triene structure by a known procedure by ketalizing the compounds of the general formula (III), hydrolyzing the ketal group of the thereby formed 5(10),9(11)-diene and reacting the 5(10),9(11)-diene-3-one obtained with dichlorodicyanobenzoquinone.

10. A pharmaceutical composition, which contains at least one compound of the general formula (I) of claims 1 to 7, optionally together with pharmaceutically tolerated inactive materials and vehicles.

11. The use of compounds of the general formula (I) of claims 1 to 7 for producing a medicament for controlling the fertility in man, for hormone replacement therapy (HRT) in men and women or for the treatment of hormone-induced diseases in men and women, such as endometriosis, breast cancer or hypogonadism.

12. Compounds of the general formula (I) of claims 1 to 7 for use as active therapeutic ingredients.

## Revendications

1. 14,15-Cyclopropanostéroïdes du groupe des 19-norandrostanes de formule générale (I) 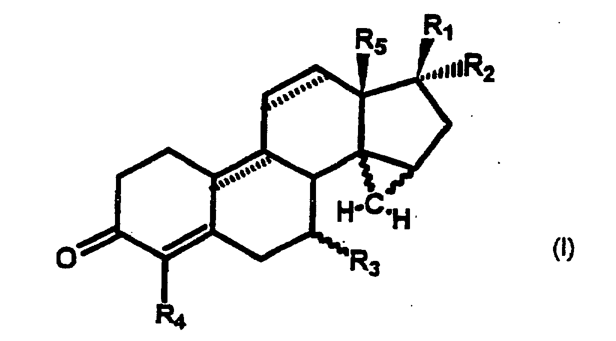
dans laquelle R₁ est un atome d'hydrogène, un groupe hydroxyle, un groupe alkyle en C₁₋₁₀, alkyloxy en C₁₋₁₀, acyloxy en C₁₋₁₅, aryloxy en C₄₋₁₅, aralkyloxy en C₇₋₁₅, ou alkylaryloxy en C₇₋₁₅,
R₂ représente un atome d'hydrogène, un groupe hydroxyle, un groupe alkyle en C₁₋₁₀, acyle en C₁₋₁₀, acyloxy en C₁₋₁₀, aryle en C₆₋₁₅, aralkyle en C₇₋₁₅, ou alkylaryle en C₇₋₁₅,
un radical -(CH₂)ₙCH₂Y,
dans lequel n = 0, 1 ou 2, et Y représente un atome d'halogène, notamment un atome de fluor, chlore, de brome ou d'iode, ou un pseudohalogène, notamment un groupe cyano, azide, ou rhodanide,
un radical -(CH₂)ₘ-CH=CH(CH₂)ₚ-R₆,
dans lequel m = 0, 1, 2 ou 3 et p = 0, 1 ou 2, et R₆ représente un atome d'hydrogène, un radical alkyle en C₁₋₁₀, aryle en C₆₋₁₅, aralkyle en C₇₋₁₅, ou alkylaryle en C₇₋₁₅, ou un groupe hydroxyle ou un groupe alkyloxy en C₁₋₁₀ ou acyloxy en C₁₋₁₀,
ou un radical -(CH₂)ₒC≡CR₇,
dans lequel o = 0, 1 ou 2 et R₇ représente un atome d'hydrogène, un atome d'halogène, notamment un atome de fluor, de chlore, de brome ou d'iode, un radical alkyle en C₁₋₁₀, aryle en C₆₋₁₅, aralkyle en C₇₋₁₅, ou alkylaryle en C₇₋₁₅, ou acyle en C₁₋₁₀,
R₁ et R₂ représentent ensemble un groupe céto, méthylène ou difluorométhylène ou forment par inclusion du C-17 un spirooxirane ou un 2,2-diméthyl-1,3-dioxolane,
R₃ représente un atome d'hydrogène ou un groupe alkyle en position α ou β,
R₄ représente un atome d'halogène, notamment un atome de fluor, de chlore, de brome oui d'iode, ou un pseudohalogène, notamment un groupe rhodanide ou azide, ou un groupe hydroxyle ou perfluoroalkyle et
R₅ représente un groupe alkyle en C₁₋₄, un groupe α- ou β-cyclopropane se trouve entre C-14 et C-15,
des doubles liaisons peuvent se trouver en position 9,10 ou 11,12,
étant entendu que, si deux doubles liaisons supplémentaires se trouvent en position 9,10 et 11,12 ou une double liaison se trouve en position 11,12, R₄, en plus des significations mentionnées précédemment, peut être un atome d'hydrogène, et étant également entendu que, si R₅ n'est pas un groupe méthyle, R₄ peut être, en plus des significations mentionnées précédemment, un atome d'hydrogène,
ainsi que leurs sels pharmaceutiquement compatibles.

2. Composés selon la revendication 1, dans lesquels R₁ représente un groupe hydroxyle ou acyloxy, notamment un groupe formyloxy, un groupe acétyloxy, un groupe propionyloxy, un groupe butyryloxy, un groupe iso-butyryloxy, un groupe heptanyloxy ou un groupe undécanyloxy.

3. Composés selon la revendication 1 ou 2, avec R₂ représentant un atome d'hydrogène ou un groupe alkyle, notamment un groupe méthyle ou éthyle.

4. Composés selon une des revendications de 1 à 3, dans lesquels R₃ est un groupe méthyle.

5. Composés selon une des revendications de 1 à 4, dans lesquels R₄ représente un atome de fluor, de chlore ou de brome, ou un groupe trifluorométhyle ou hydroxy.

6. Composés selon une des revendications de 1 à 5, dans lesquels R₅ représente un groupe méthyle ou éthyle.

7. Composés selon la revendication 1, à savoir
1) 4-chloro-17β-hydroxy-14α,15α-méthylène-estr-4-én-3-one,
2) 4-chloro-17α-hydroxy-14α,15α-méthylène-estr-4-én-3-one,
3) 4-chloro-17β-hydroxy-14β,15β-méthylène-estr-4-én-3-one,
4) 4-chloro-17α-hydroxy-14β,15β-méthylène-estr-4-én-3-one,
5) 4-bromo-17β-hydroxy-14α,15α-méthylène-estr-4-én-3-one,
6) 4-bromo-17α-hydroxy-14α,15α-méthylène-estr-4-én-3-one,
7) 4-bromo-17β-hydroxy-14β,15β-méthylène-estr-4-én-3-one,
8) 4-bromo-17α-hydroxy-14β,15β-méthylène-estr-4-én-3-one,
9) 4-fluoro-17β-hydroxy-14α,15α-méthylène-estr-4-én-3-one,
10) 4-fluoro-17α-hydroxy-14α,15α-méthylène-estr-4-én-3-one,
11) 4-fluoro-17β-hydroxy-14β,15β-méthylène-estr-4-én-3-one,
12) 4-fluoro-17α-hydroxy-14β,15β-méthylène-estr-4-én-3-one,
13)4,17β-dihydroxy-14α,15α-méthylène-estr-4-én-3-one,
14) 4,17α-dihydroxy-14α,15α-méthylène-estr-4-én-3-one,
15) 4,17β-dihydroxy-14β,15β-méthylène-estr-4-én-3-one,
16) 4,17α-dihydroxy-14β,15β-méthylène-estr-4-én-3-one,
17) 4-trifluorométhyl-17β-hydroxy-14α,15α-méthylène-estr-4-én-3-one,
18) 4-trifluorométhyl-17α-hydroxy-14α,15α-méthylène-estr-4-én-3-one,
19) 4-trifluorométhyl-17β-hydroxy-14β,15β-méthylène-estr-4-én-3-one,
20) 4-trifluorométhyl-1 7α-hydroxy-14β,15β-méthylène-estr-4-én-3-one,
22) 17β-hydroxy-14α,15α-méthylène-estra-4,9,11-trién-3-one,
23) 17α-hydroxy-14α,15α-méthylène-estra-4,9,11-trién-3-one,
24) 17β-hydroxy-14α,15α-méthylène-estra-4,9,1 1-trién-3-one,
25) 17β-hydroxy-14β,15β-méthylène-estra-4,9,11-trién-3-one et
26) 17α-hydroxy-14β,15β-méthylène-estra-4,9,11-trién-3-one.

8. Procédé de préparation des composés selon une des revendications de 1 à 7, **caractérisé en ce que** dans un 14,15-cyclopropanostéroïde de formule générale (II) 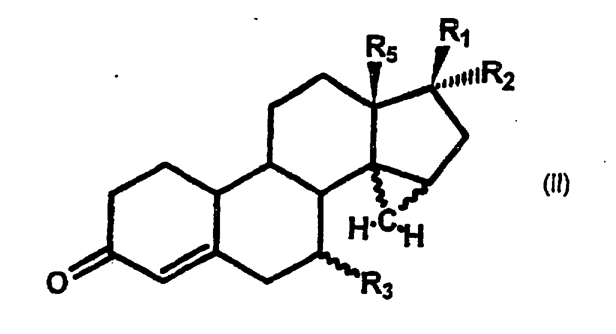 dans laquelle R₁, R₂, R₃, et R₅ ont la signification mentionnée dans les revendications de 1 à 7, on époxyde la double liaison 4,5 avec du peroxyde d'hydrogène dans des conditions alcalines, on traite le mélange époxyde obtenu dans un solvant approprié avec des acides de formule générale HR₈,
dans laquelle R₈ peut être un atome d'halogène, notamment un atome de fluor, de chlore ou de brome, un pseudohalogène, notamment un groupe azide ou rhodano, ou on le transforme avec des quantités catalytiques d'un acide minéral, et on transforme éventuellement les composés 4-bromo obtenus précédemment de formule générale (I) avec du méthylester d'acide difluoro-2-(fluorosulfonyl)acétique dans le diméthylformamide en présence de Cul.

9. Procédé de préparation de composés selon une des revendications de 1 à 7, **caractérisé en ce qu'**on transforme des composés de formule générale (III), 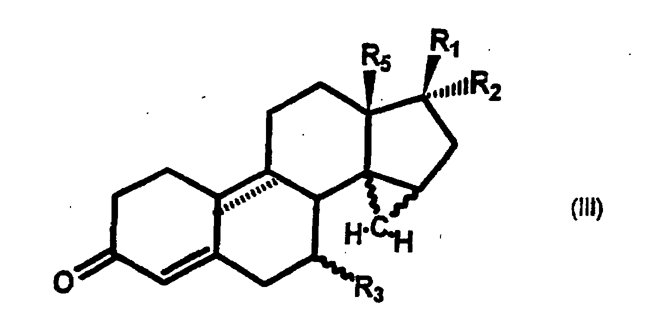 dans laquelle R₁, R₂, R₃, et R₅ ont la signification mentionnée dans les revendications de 1 à 7, d'une manière connue en soi, en effectuant une cétalisation des composés de formule générale (III), une hydrolyse du groupe cétal du 5(10),9(11)-diène ainsi obtenu et une conversion du 5(10),9(11)-dién-3-one obtenu avec de la dichlorodicyanobenzoquinone en composés souhaités de formule générale (I) ayant une structure de 4,9,11-triène.

10. Composition pharmaceutique contenant au moins un composé de formule générale (I) selon les revendications de 1 à 7, éventuellement associé à des adjuvants et des agents de support pharmaceutiquement compatibles.

11. Utilisation des composés de formule générale (I) selon les revendications de 1 à 7 pour préparer un médicament destiné au contrôle de la fertilité chez l'homme, à l'Hormone-Therapy-Replacement (HRT) chez l'homme et la femme ou au traitement des maladies hormonales chez l'homme et la femme comme par exemple l'endométriose, le cancer du sein ou l'hypogonadisme.

12. Composés de formule générale (I) selon les revendications 1 à 7 destinés à une application en tant qu'agents actifs thérapeutiques.
